(19) Europäisches Patentamt
European Patent Office
Office européen des brevets

(11) Veröffentlichungsnummer: **0 078 462**
**B1**

(12) # EUROPÄISCHE PATENTSCHRIFT

(45) Veröffentlichungstag der Patentschrift: **15.01.86**

(51) Int. Cl.⁴: **C 07 D 285/00**, C 07 F 9/65, A 01 N 43/72, A 01 N 57/24

(21) Anmeldenummer: **82109745.8**

(22) Anmeldetag: **22.10.82**

(54) **1,2,4,6-Thiatriazin-1,1-dioxide, Verfahren zu ihrer Herstellung und ihre Verwendung zur Bekämpfung unerwünschten Pflanzenwuchses.**

(30) Priorität: **02.11.81 DE 3143381**

(43) Veröffentlichungstag der Anmeldung: **11.05.83 Patentblatt 83/19**

(45) Bekanntmachung des Hinweises auf die Patenterteilung: **15.01.86 Patentblatt 86/3**

(84) Benannte Vertragsstaaten: **BE CH DE FR GB IT LI NL**

(56) Entgegenhaltungen:
**EP-A-0 029 908**
**EP-A-0 039 426**
**EP-A-0 075 117**
**DE-A-2 508 832**
**DE-A-2 933 889**

**CHEMICAL ABSTRACTS; Band 92, Nr. 9, 3. März 1980, Seiten 376,377, Nr. 76465a, Columbus, Ohio, USA, S. KARADY et al.: "Heterocycles from chlorosulfonyl isocyanate II. Reaction with isothioureas"**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Acker, Rolf-Dieter, Dr., Tuchbleiche 8, D-6906 Leimen (DE)**
Erfinder: **Sauter, Hubert, Dr., Neckarpromenade, D-6800 Mannheim (DE)**
Erfinder: **Theobald, Hans, Dr., Parkstrasse 2, D-6703 Limburgerhof (DE)**
Erfinder: **Wuerzer, Bruno, Dr. Dipl.-Landwirt, Ruedigerstrasse13, D-6701 Otterstadt (DE)**

**EP 0 078 462 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft 1,2,4,6-Thiatriazin-1,1-dioxide, Verfahren zu ihrer Herstellung, Herbizide, die diese Verbindungen als Wirkstoffe enthalten, und ihre Verwendung zur Bekämpfung unerwünschten Pflanzenwuchses.

Es ist bekannt, daß substituierte 6H-1,2,4,6-Thiatriazin-(5)-on-1,1-dioxidderivate eine herbizide Wirkung haben (DE-OS 25 08 832, DE-OS 29 33 889). Außerdem sind 1,2,4,6-Thiatriazin-1,1-dioxide mit herbizider Wirkung Gegenstand der EP-A-75117. Diese Verbindungen tragen u.a. in 3-Stellung einen substituierten Benzylrest und in 3-Stellung einen aliphatischen Rest.

Es wurde gefunden, daß 1,2,4,6-Thiatriazin-1,1-dioxide der Formel

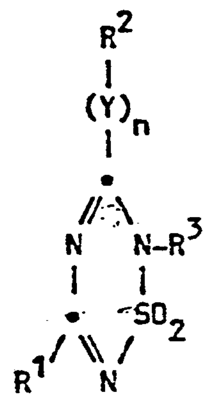

(I),

in der

$R^1$ Wasserstoff, einen unverzweigten oder verzweigten Alkyl- oder Alkenylrest mit bis zu 10 Kohlenstoffatomen, Propargyl, But-1-in-3-yl, But-2-inyl, einen durch Halogen substituierten, gesättigten, unverzweigten oder verzweigten aliphatischen Rest mit bis zu 10 Kohlenstoffatomen, 2-Methoxyethyl,.2-Ethoxyethyl, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Meth-oxy-n-butyl, 4-Methoxy-n-butyl, 2-Methylmercapto-ethyl, 2-Ethylmercapto-ethyl, 3-Methylmercapto-n- propyl, 3-Methylmercapto-n-butyl, 1-Methylmercapto-set.-butyl, Methylmercapto-tert.-butyl, 2-Methylmercapto-n-butyl, einen Alkylamino- oder Dialkylaminorest mit 1 bis 6 Kohlenstoffatomen in einer Alkylgruppe, einen Cycloalkylrest mit 3 bis 7 Kohlenstoffatomen, einen gegebenenfalls durch Halogen, Alkyl oder Alkoxy mit 1 bis 4 Kohlenstoffatomen substituierten Phenylrest, einen gegebenenfalls durch Halooen substituierten Benzylrest oder den Rest $R^4$-X-, wobei $R^4$ die für $R^1$ genannten Bedeutungen, ausgenommen Alkylamino- und Dialkylamino, hat und X Sauerstoff, Schwefel, -SO- oder -$SO_2$- bedeutet,

$R^2$ einen durch Cyano, Thiocyano, Nitro oder Vinylketo substituierten gesättigten oder ungesättigten, unverzweigten oder verzweigten aliphatischen Rest mit bis zu 10 Kohlenstoffatomen, 2-Allyloxyethyl, 2-propargyloxyethyl, 2-Allyloxy-n-propyl, 2-Propargyloxy-n-propyl, 3-Allyloxy-n-propyl, 4-Allyloxy-but-2-inyl, 2-Allylmercaptoethyl, 2-Propargylmercaptoethyl, 2-Allylmercapto-n-propyl, einen durch Alkyl, Alkenyl oder Alkinyl mit jeweils bis zu 4 Kohlenstoffatomen substituierten Alkylenglykolalkyl-, -alkenyl- oder alkinylrest mit bis zu 6 Kohlenstoffatomen, einen durch Alkoxycarbonyl oder Alkylmercaptocarbonyl mit bis zu 5 Kohlenstoffatomen substituierten Alkenyl- oder Alkinylrest mit jeweils bis zu 4 Kohlenstoffatomen, einen Alkylidenamino- oder Dialkylmethylidenaminorest mit bis zu 6 Kohlenstoffatomen, einen gegebenenfalls durch Halogen, Alkyl, Halogenalkyl, Alkoxy, Halogenalkoxy, Alkylmercapto oder Halogenalkylmercapto mit jewells bis zu 4 Kohlenstoffatomen, Nitro, Cyano, Thiocyano am Phenylring substituierten Phenylalkyl-, Phenylalkenyl- oder Phenylalkinylrest mit 8 bis 12 Kohlenstoffatomen,

$R^3$ Wasserstoff, einen unverzweigten oder verzweigten Alkyloder Alkenylrest mit bis zu 10 Kohlenstoffatomen, Propargyl, But-1-in-3-yl, But-2-inyl, einen durch Halogen substituierten gestättigten, unverzweigten oder verzweigten aliphatischen Rest mit bis zu 10 Kohlenstoffatomen, 2-Methoxyethyl, 2-Ethoxyethyl, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Methoxy-n-butyl, 4-Methoxy-n-butyl, oder einen Cycloalkylrest mit 3 bis 7 Kohlenstoffatomen und

Y Sauerstoff, Schwefel, -SO- oder -$SO_2$- und

n 1 bedeuten,

oder in der $R^2$ einen durch Nitro oder Halogen und Nitro substituierten Benzylrest oder einen durch Halogen, Alkyl, Halogenalkyl, Alkoxy, Halogenalkoxy, Alkylmercapto oder Halogenalkylmercapto mit jeweils bis zu 4 Kohlenstoffatomen, Nitro, Cyano, Thiocyano am Phenylring substituierter Phenoxyalkyl-, Phenoxyalkenyl- oder Phenoxyalkinylrest mit 8 bis 12 Kohlenstoffatomen bedeutet, wenn $R^1$ den Rest $R^4$-X- und n 1 bedeuten, oder in der $R^2$ einen Dialkylphosphonorest bedeutet, wenn n 0 ist, eine gute herbizide Wirkung haben.

In der Formel stehen $R^1$ und $R^3$ für Wasserstoff, einen unverzweigten oder verzweigten Alkyl- oder Alkenylrest mit bis zu 10 Kohlenstoffatomen, vorzugsweise mit bis zu 4 Kohlenstoffatomen, wie Methyl, Ethyl, n-Propyl,

Isopropyl, n-Butyl, Isobutyl, sec.-Butyl, tert.-Butyl, n-Pentyl, tert.-Amyl, n-Hexyl, Pentyl-3, 1,2-Dimethyl-n-propyl, 1,3-Dimethyl-n-butyl, 1-Ethyl-2-methyl-n-propyl, 1,2,2-Tri-methyl-n-propyl, 1,2-Dimethyl-4-hexyl, Allyl, Methallyl, Crotyl, 2-Ethyl-hex-2-enyl, Hex-5-enyl, 2-Methyl-but-2-enyl, 2-Methyl-but-3-enyl, But-1-en-3-yl, 2-Methyl-but-1-en-4-yl, 2-Methyl-but-2-en-4-yl, 3-Methyl-but-1-en-3-yl, für Propargyl, But-1-in-3-yl, But-2-inyl, für einen durch Halogen substituierten gesättigten, unverzweigten oder verzweigten aliphatischen Rest mit bis zu 10 Kohlenstoffatomen, vorzugsweise mit bis zu 4 Kohlenstoffatomen, beispielsweise einen Halogenalkylrest mit bis zu 10 Kohlenstoffatomen, vorzugsweise mit 1 bis 4 Kohlenstoffatomen, wie 2-Chlorethyl, 2-Chlor-n-propyl, 3-Chlor-n-propyl, 2-Chlor-sec.-butyl, 2-Chlor-isobutyl, 2-Fluor-sec.-butyl, 2-Fluor-isobutyl, 2-Fluor-isopropyl, Chlor-tert.-butyl, 2,2,2-Trifluorethyl, oder für 2-Methoxyethyl, 2-Ethoxyethyl, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Methoxy-n-butyl, 4-Methoxy-n-butyl, oder für einen Cycloalkylrest mit 3 bis 7 Kohlenstoffatomen, wie Cyclopropyl, Cyclopentyl, Cyclohexyl.

$R^1$ steht außerdem für einen Alkylamino- oder Dialkylaminorest mit 1 bis 6 Kohlenstoffatomen, vorzugsweise mit 1 bis 4 Kohlenstoffatomen, in einer Alkylgruppe, wie Methylamino, Dimethylamino, Ethylamino, Isopropylamino, n-Butylamino, Methylethylamino, Diisopropylamino, für 2-Methylmercapto-ethyl, 2-Ethylmercapto-ethyl, 3-Methylmercapto-n-propyl, 3-Methylmercapto-n-butyl, 1-Methylmercapto-sec.-butyl, Methylmercapto-tert.-butyl, 2-Methylmercapto-n-butyl oder für einen gegebenenfalls durch Halogen, Alkyl oder Alkoxy mit 1 bis 4 Kohlenstoffatomen substituierten Phenylrest oder für einen gegebenenfalls durch Halogen am Phenylring substituierten Benzylrest, wie Phenyl, 4-Chlorphenyl, 3,4-Dichlorphenyl, o-, m-, p-tert.-Butylphenyl, o-, m-, p-Methoxyphenyl, o-, m-, p-Methylphenyl, 4-Methoxy-3-chlor-phenyl, 2-Methyl-4-chlorphenyl, Benzyl, 2,6-Dichlorbenzyl, 2-Chlor-6-fluorbenzyl, 2,6-Difluorbenzyl, o-, m-, p-Chlorbenzyl.

$R^1$ kann außerdem den Rest $R^4$ -X-bedeuten, wobei $R^4$ die für $R^1$ genannten Bedeutungen, ausgenommen Alkylamino- und Dialkylamino, hat und X Sauerstoff, Schwefel, -SO- oder,$SO_2$-, vorzugsweise Sauerstoff oder Schwefel, bedeutet.

$R^2$ in Formel I steht für einen durch Cyano, Thiocyano, Nitro oder Vinylketo substituierten gesättigten oder ungesättigten, unverzweigten oder verzweigten aliphatischen Rest mit bis zu 10 Kohlenstoffatomen, vorzugsweise mit bis zu 4 Kohlenstoffatomen, wie 2-Cyanoethyl, 2-Cyano-n-propyl, 3-Cyano-n-propyl, 4-Cyano-n-butyl, 3-Cyano-prop-2-enyl, 4-Cyano-but-2-inyl, 2-Nitroethyl, 2-Nitro-n-propyl, 3-Nitro-n-propyl, 3-Nitro-prop-2-enyl, Vinylketoethyl, 2-Thiocyanoethyl, 2-Thiocyano-n-propyl, 3-Thiocyano-n-propyl, 4-Thiocyano-n-butyl, 4-Thiocyano-but-2-inyl, für 2-Allyloxyethyl, 2-Propargyloxyethyl, 2-Allyloxy-n-propyl, 2-Propargyloxy-n-propyl, 3-Allyloxy-n-propyl, 4-Allyloxy-but-2-inyl, 2-Allyl-mercaptoethyl, 2-Propargylmercaptoethyl, 2-Allylmercapto-n-propyl, für einen durch Alkyl, Alkenyl oder Alkinyl mit jeweils bis zu 4 Kohlenstoffatomen substituierten Alkylenglykolalkylrest mit bis zu 6 Kohlenstoffatomen, vorzugsweise mit bis zu 4 Kohlenstoffatomen, wie 2-(Methoxyglykol)-ethyl, 2-(Allyloxy-glykol)-ethyl, 2-(Propargyloxy-glykol)-ethyl, 3-(Methoxyglykol)-n-propyl, 4-(Methoxyglykol)-n-butyl, für einen durch Alkoxycarbonyl oder Alkylmercaptocarbonyl mit jeweils bis zu 5 Kohlenstoffatomen substituierten Alkenyl- oder Alkinylrest mit jeweils bis zu 4 Kohlenstoffatomen, wie 3-Methoxycarbonyl-prop-2-enyl, 3-Ethoxycarbonyl-prop-2-enyl, 3-n-Propoxycarbonyl-prop-2-enyl, 3-n-Butoxycarbonyl-prop-2-enyl, 3-Methoxycarbonyl-prop-2-inyl, 3-Ethoxycarbonyl-prop-2-inyl, 3-n-Butoxycarbonyl-prop-2-inyl, 3-Methylmercaptocarbonyl-prop-2-enyl, für einen Alkylidenamino- oder Dialkylmethylidenaminorest mit bis zu 6 Kohlenstoffatomen, wie Ethylidenamino, n-Propylidenamino, n-Butylidenamino, Prop-2-ylidenamino, But-2-ylidenamino, Pent-2-ylidenamino, Pent-3-ylidenamino, Hex-3-ylidenamino, für einen gegebenenfalls durch Halogen, Alkyl, Halogenalkyl, Alkoxy, Halogenalkoxy, Alkylmercapto oder Halogenalkylmercapto mit jeweils bis zu 4 Kohlenstoffatomen, Cyano, Nitro, Thiocyano am Phenylring substituierten Phenylalkyl- Phenylalkenyl- oder Phenylalkinylrest mit 8 bis 12 Kohlenstoffatomen, wie 2-(o-, m-, p-Chlorphenyl)-ethyl, 2-(2′, 4′-Dichlorphenyl)-ethyl, 2-(2′, 4′, 6′-Trichlorphenyl)-ethyl, 2-(3′, 5′-Dichlorphenyl)-ethyl, 2-(o-, m-, p-Tolyl)-ethyl, 2-(3′, 4′-Xylol)-ethyl, 2-(o-, m-, p-Trifluormethylphenyl)-ethyl, 2-(o-, m-, p-Anisyl)-ethyl, 2-(o-, m-, p-Difluormethoxyphenyl)-ethyl, 2-(o-, m-, p-Chlordifluormethoxyphenyl)-ethyl, 2-(o-, m-, p-Methylmercaptophenyl)-ethyl, 2-(o-, m-, p-Trifluormethyl-mercaptophenyl)-ethyl, 2-(o-, m-, p-Cyanophenyl)-ethyl, 2-(o-, m-, p-Thiocyanatophenyl)-ethyl, 3-(o-, m-, p-Chlorphenyl)-n-propyl, 3-(o-, m-, p-Trifluormethylphenyl)-n-propyl, 2-Phenyl-ethyl, 3-Phenyl-n-propyl, 4-Phenyl-n-butyl, 5-Phenyl-n-pentyl, 2-(o-, m-, p-Nitrophenyl)-ethyl, 2-(3′-Chlor-4′-methoxy-phenyl)-ethyl, 2-(4′-Chlor-3′-methoxyphenyl)-ethyl, 2-(2′-Chlor-4′-trifluormethylphenyl)-ethyl, 2-Phenyl-ethylen, 2-(o-, m-, p-Chlorphenyl)-ethylen, 2-(o-, m-, p-Tolyl)-ethylen, 2-(o-, m-, p-Nitrophenyl)-ethylen, 3-Phenyl-prop-2-enyl, 3-(o-, m-, p-Chlorphenyl)-prop-2-enyl, 3-(o-, m-, p-Tolyl)-prop-2-enyl, 3-(o-, m-, p-Nitrophenyl)-prop-2-enyl oder, falls n 0 bedeutet, für einen Dialkylphosphonorest der Formel

$$(ALKO)_2\overset{\overset{\displaystyle O}{\|}}{P}-$$

mit jeweils 1 bis 4 Kohlenstoffatomen in einer Alkylgruppe, wie Dimethylphosphono, Methyl-ethylphosphono, Methyl-n-propylphosphono.

$R^2$ steht außerdem, falls $R^1$ die Bedeutung von $R^4$-X- hat, für einen durch Nitro oder Halogen und Nitro substituierten Benzylrest, wie o-, m-, p-Nitrobenzyl, 2-Chloro-6-nitrobenzyl, 4-Chlor-6-nitrobenzol, 4-Nitro-6-chlorbenzyl, oder einen durch Halogen, Alkyl, Halogenalkyl, Alkoxy, Halogenalkoxy, Alkylmercapto oder Halogenalkylmercapto mit jeweils bis zu 4 Kohlenstoffatomen, Nitro, Cyano, Thiocyano am Phenylring

substituierten Phenoxyalkyl-, -alkenyl- oder -alkinylrest mit bis zu 12 Kohlenstoffatomen, vorzugsweise mit bis zu 10 Kohlenstoffatomen, wie 2-(o-,m-,p-Chlorphenoxy)-ethyl, 2-(2' 4'-Dichlor-phenoxy)-ethyl, 2-(2', 4', 6'-Trichlorphenoxy)-ethyl, 2-(3', 5'-Dichlorphenoxy)-ethyl, 2-(3', 4'-Dichlorphenoxy)-ethyl, 2-(o-,m-,p-Kresoxy)-ethyl, 2-(3', 4'-Dimethylphenoxy)-ethyl, 2-(o-,m-,p-Trifluormethylphenoxy)-ethyl, 2-(o-,m-,p-Methoxyphenoxy)-ethyl, 2-(o-,m-,p-Difluor-methoxyphenoxy)-ethyl, 2-(o-,m-,p-Chlordifluormethoxy-phenoxy)-ethyl, 2-(o-,m-,p-Methylmercaptophenoxy)-ethyl, 2-(o-,m-,p-Cyano-phenoxy)-ethyl, 2-(o-,m-,p-Thiocyanato-phenoxy)-ethyl, 3-(o-,m-,p-Chlorphenoxy)-n-propyl, 3-(o-,m-,p-Trifluormethylphenoxy)-n-propyl, 2-Phenoxy-ethyl, 3-Phenoxy-n-propyl, 4-Phenoxy-n-butyl, 5-Phenoxy-n-butyl, 2-(o-,m-,p-Nitrophenoxy)-ethyl, 2-(3'-Chlor-4'-methoxyphenoxy)-ethyl, 2-(2'-Chlor-4'-trifluormethyl-phenoxy)-ethyl, Phenoxy-ethylen, 2-(o-,m-,p-Chlorphenoxy)-ethylen, 2-(o-,m-,p-Kresoxy)-ethylen, 2-(o-,m-,p-Nitrophenoxy)-ethylen, 2-Phenoxy-prop-2-enyl, 3-(o-,m-,p-Chlorphenoxy)prop-2-enyl, 3-(o-,m-,p-Kresoxy)-prop-2-enyl, 3-(o-,m-,p-Nitrophenoxy)-prop-2-enyl.

Bevorzugte Verbindungen der Formel I sind solche, bei denen $R^1$ für den Rest $R^4$-X-, wobei $R^4$ Alkyl mit 1 bis 4 Kohlenstoffatomen und X Sauerstoff bedeutet, $R^2$ für einen Dialkylphosphonorest mit jeweils 1 bis 4 Kohlenstoffatomen in einer Alkylgruppe und $R^3$ für Alkyl mit 1 bis 4 Kohlenstoffatomen stehen und n 0 bedeutet, oder solche, bei denen $R^1$ den Rest $R^4$-X- bedeutet, wobei $R^4$ Alkyl mit 1 bis 4 Kohlenstoffatomen und X Sauerstoff oder Schwefel sind, und $R^2$ einen durch Halogen, Cyano, Nitro oder Alkyl am Phenylring substituierten Phenoxyalkylrest mit 7 bis 10 Kohlenstoffatomen, $R^3$ für Alkyl mit 1 bis 4 Kohlenstoffatomen und Y für Sauerstoff oder Schwefel stehen, oder solche, bei denen $R^1$ und $R^3$ Alkyl mit 1 bis 4 Kohlenstoffatomen, $R^2$ einen durch Cyano substituierten gesättigten oder ungesättigten aliphatischen Rest mit bis zu 4 Kohlenstoffatomen, vorzugsweise einen Alkylrest, und Y Sauerstoff oder Schwefel, oder solche, bei denen $R^1$ den Rest $R^4$-X-, wobei $R^4$ Alkyl mit 1 bis 4 Kohlenstoffatomen und X Sauerstoff oder Schwefel bedeutet, $R^2$ einen durch Cyano substituierten gesättigten oder ungesättigten aliphatischen Rest mit bis zu 4 Kohlenstoffatomen, vorzugsweise einen Alkylrest, $R^3$ Alkyl mit 1 bis 4 Kohlenstoffatomen und Y Sauerstoff oder Schwefel bedeutet, oder solche, bei denen $R^1$ und $R^3$ Alkyl mit 1 bis 4 Kohlenstoffatomen, $R^2$ einen durch Halogen, Cyano, Nitro oder Alkyl, vorzugsweise durch Halogen, am Phenylring substituierten Phenylalkyl-, Phenylalkenyl- oder Phenylalkinylrest mit 8 bis 12 Kohlenstoffatomen und Y Sauerstoff oder Schwefel bedeuten.

Man erhält die 1, 2, 4, 6-Thiatriazin-1, 1-dioxide der Formel 1 durch Umsetzung von Verbindungen der Formel

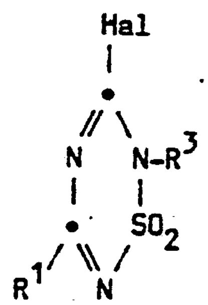 (II),

in der $R^1$ und $R^3$ die obengenannten Bedeutungen haben und Hal Halogen bedeutet, mit einer Verbindung der Formel

$$H - (Y)_n - R^2 \qquad (III),$$

in der $R^2$, Y und n die obengenannten Bedeutungen haben,

oder einem Alkali-, Erdalkali- oder Ammoniumsalz, einer Verbindung der Formel III, gegebenenfalls in einem inerten organischen Lösungsmittel und gegebenenfalls in Anwesenheit eines Säureakzeptors bei einer Temperatur zwischen -50 und +150°C. Die Umsetzung kann drucklos oder unter Druck kontinuierlich oder diskontinuierlich durchgeführt werden.

Verwendet man 3-Chlor-5-methyl-2-isopropyl-2H-1, 2, 4, 6-thiatriazin-1, 1-dioxid und β-Hydroxypropionitril als Ausgangsmaterialien, so kann der Reaktionsablauf durch folgendes Formelschema wiedergegeben werden:

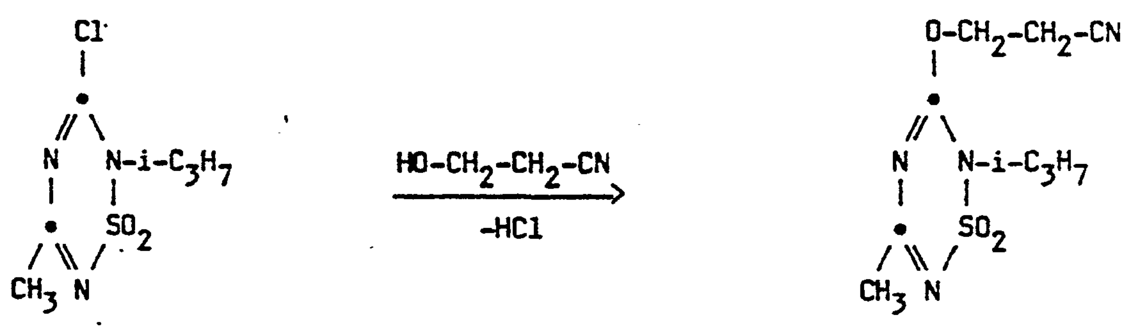

Verwendet man 5-Chlor-6-isopropyl-3-methoxy-6H-1, 2, 4, 6-thiatriazin-1, 1-dioxid und 2-(p-Chlorphenyl)-ethanol als Ausgangsmaterialien, so kann der Reaktionsablauf durch folgendes Formelschema wiedergegeben werden:

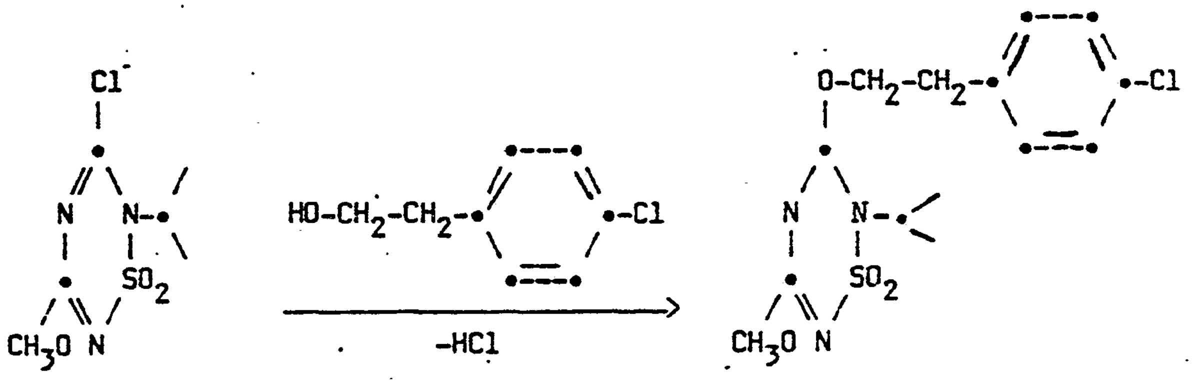


Verwendet man 5-Chlor-6-methyl-3-methylmercapto-6H-1,2,4,6-thiatriazin-1,1-dioxid und Trimethyl-phosphit als Ausgangsmaterialien, so kann der Reaktionsablauf durch folgendes Formelschema wiedergegeben werden:

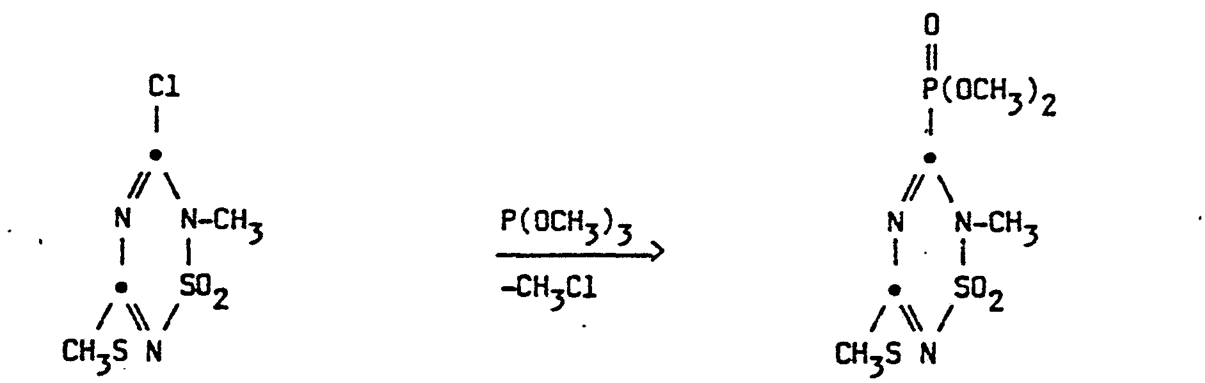


Zweckmäßigerweise verwendet man für die Umsetzung unter den jeweiligen Reaktionsbedingungen inerte Lösungs- oder Verdünnungsmittel. Als Lösungsmittel kommen beispielsweise Halogenkohlenwasserstoffe, insbesondere Chlorkohlenwasserstoffe, z.B. Tetrachlorethylen-1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-,p-Difluorbenzol, 1, 2-Di-chlorethan, 1, 1-Dichlorethan, 1, 2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1, 2, 4-Trichlorbenzol; Ether, z.B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, β, β'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2, 2, 4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Zweckmäßig verwendet man das Lösungsmittel in einer Menge von 100 bis 2.000 Gew.%, vorzugsweise von 200 bis 700 Gew.%, bezogen auf Ausgangsstoff II.

Als Säureakzeptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. als basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformfat, Natriumacetat, Trimethylamin, Triethylamin, Tripropylamin, Triisopropylamin, Tributylamin,

Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßigerweise verwendet man den Säureakzeptor in einem Über- oder Unterschuß von bis zu 20 %, bezogen auf den Ausgangsstoff II. Man kann jedoch auch den entstehenden Halogenwasserstoff durch Eingasen eines Inertgases, beispielsweise von Stickstoff, entfernen.

Die zur Umsetzung benötigten Ausgangsstoffe III werden im allgemeinen in einem Über- oder Unterschuß von bis zu 20 %, bezogen auf den Ausgangsstoff II, eingesetzt. Man kann den Ausgangsstoff III jedoch auch direkt als Lösungsmittel verwenden. Man kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Zweckmäßigerweise wird das Verfahren zur Herstellung der neuen Verbindungen so durchgeführt, daß man den Ausgangsstoff II, gegebenenfalls in einem der vorgenannten Verdünnungsmittel vorlegt und dann den Ausgangsstoff III und einen Säureakzeptor gleichzeitig oder nacheinander zugibt.

Die Umsetzung ist in vielen Fällen nach der Zugabe der Komponenten bereits abgeschlossen, andernfalls rührt man zu ihrer Beendigung noch 10 Minuten bis 10 Stunden bei -50 bis 150°C, vorzugsweise 0 bis 120°C, insbesondere 10 bis 50°C, nach.

Verwendet man ein Inertgas zur Entfernung des Halogenwasserstoffes, so rührt man zweckmäßigerweise 0,2 bis 10 Stunden bei 40 bis 100°C nach.

Aus dem Reaktionsgemisch wird der Endstoff I in üblicher Weise, z.B. nach Abdestillieren von Lösungsmittel oder überschüssigem Ausgangsstoff III oder direkt durch Absaugen, isoliert. Der verbleibende Rückstand wird in diesem Fall zur Entfernung saurer Verunreinigungen mit Wasser bzw. verdünntem Alkali gewaschen und getrocknet. Im Falle von mit Wasser nicht mischbaren Verdünnungsmitteln kann man auch direkt das Reaktionsgemisch mit Wasser bzw. mit verdünntem Alkali extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation gereinigt werden.

Man erhält die Ausgangsverbindungen der Formel II durch Umsetzung von Verbindungen der Formel

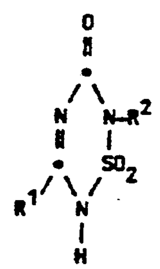 (IV),

in der

$R^1$ und $R^2$ die im Anspruch 1 genannten Bedeutungen haben, mit der Maßgabe, daß $R^1$ nicht den Rest $R^4$-X-bedeutet, oder deren Alkalimetallsalzen oder Erdalkalimetallsalzen mit einem Säurehalogenid der Phosphorsäure, phosphorigen Säure, Kohlensäure, Oxalsäure oder schwefligen Säure gegebenenfalls in Gegenwart eines Lösungs- oder Verdünnungsmittels und gegebenenfalls in Gegenwart eines Reaktionsbeschleunigers.

Zweckmäßigerweise verwendet man für die Umsetzung unter den Reaktionsbedingungen inerte Lösungs- oder Verdünnungsmittel, wie Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Tetrachlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, o-, m-, p-Dichlorbenzol, o-, m-, p-Dibrombenzol, o-, m-, p-Chlortoluol, 1,2,4-Trichlorbenzol, oder Ether, z.B. Ethylpropylether, Methyl-tert.-butylether, n-Butylether, Diisopropylether, Anisol, Dioxan, Ethylenglykoldimethylether, oder Nitro-kohlenwasserstoffe, z.B. Nitrobenzol, o-, m-, p-Chlornitrobenzol, o-Nitrotoluol, oder Nitrile, z.B. Acetonitril, Butyronitril, Isobutyronitril, Benzonitril, m-Chlorbenzonitril, oder aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, oder Ester, z.B. Ethylacetat, und entsprechende Gemische. Geeignete Lösungsmittel sind ferner anorganische

Säurechloride, z.B. Phosphoroxychlorid, oder entsprechende Mischungen mit inerten Chlorkohlenwasserstoffen, wie 1,2-Dichlorethan. Zweckmäßigerweise verwendet man das Lösungsmittel in einer Menge von 100 bis 2000 Gew.%, vorzugsweise von 200 bis 700 Gew.%, bezogen auf den Ausgangsstoff der Formel IV.

Bevorzugte Säurehalogenide sind Thionylchlorid, Schwefeltetrafluorid, Phosgen, Oxalsäurechlorid, Phosphortribromid und insbesondere Phosphorpentachlorid, Phosphortrichlorid und Phosphoroxichlorid. Die Umsetzung wird im allgemeinen mit eine, Menge von 1,0 bis 1,5, vorzugsweise 1,05 bis 1,2 Mol, Säurehalogenid, bezogen auf Ausgangsstoff IV, durchgeführt; im Falle der Verwendung fünfwertiger Phosphorhalogenverbindungen verwendet man 0,7 bis 1,5, vorzugsweise 1,0 bis 1,2 Mol, Phosphorpentahalogenid, bezogen auf Ausgangsstoff IV.

Im Falle der Verwendung eines Phosphor(V)-halogenids als Halogenierungsagens empfiehlt sich die Verwendung eines Phosphoroxihalogenids als Verdünnungsmittel, das zweckmäßigerweise in einer Menge von 1 bis 10 Mol Phosphoroxihalogenid, bezogen auf Ausgangsstoff IV, eingesetzt wird.

Hierbei kann das Phosphor(V)-halogenid auch direkt in situ hergestellt werden, indem man beispielsweise eine Mischung eines Phosphor(III)-halogenids in Phosphoroxihalogenid oder einem der obengenannten inerten Lösungsmittel mit der erforderlichen stöchiometrischen Menge aktivem Halogen umsetzt, beispielsweise nach dem in der US-PS 1 906 440 beschriebenen Verfahren, und dann nach Zugabe des Ausgangsstoffes IV die Umsetzung durchführt.

Als Reaktionsbeschleuniger kann man vorteilhafterweise ein am Stickstoffatom disubstituiertes, gegebenenfalls cyclisches Carbonsäureamid, einen tetraalkylsubstituierten Harnstoff, ein tertiäres Amin, in einer Menge von 1 bis 10 Gew.%, bezogen auf den Ausgangsstoff IV, verwenden. Auch Gemisch der genannten Katalysatoren kommen für die Reaktion in Betracht. Ferner kann man auch Salze von Diaminen, z.B. die Hydrochloride der Amine, oder ein quaternäres Salz eines Amins verwenden. Bevorzugte Katalysatoren sind:

Triethylamin, Pyridin, N,N-Dimethylanilin, N-Ethylpiperidin, N-Methylpyrrolidin, α, β- oder γ-Picolin, Chinolin, Isochinolin, Chinazolin, Chinoxalin, N-Propyl-diisopropylamin,-2,6- und 2,4-Lutidin, N-(4-Pyridyl)-pyridiniumchlorid-hydrochlorid, p-Dimethylaminopyridin, Pyrimidin, Acridin, Dimethylformamid, Diethylformamid, Ameisensäure-N-methylanilid, N,N-Dimethylacetamid, N-Methylpyrrolidon, Tetramethylharnstoff.

Die als Ausgangsstoffe der Formel IV benötigten 2H-1,2,4,6-Thiatriazin(3)on-1,1-dioxide sind teilweise bekannt oder lassen sich durch Umsetzung von N-Carbo-alkoxy-amidinen mit Aminosulfonylhalogeniden herstellen.

Man erhält beispielsweise 2-Ethyl-5-methyl-3-chlor-2H-1,2,4,6-thiatriazin-1,1-dioxid, indem man 225 Teile 2-Ethyl-5-methyl-2H-1,2,4,6-thiatriazin(3)on-1,1-dioxid innerhalb 2 Minuten unter Rühren in eine Mischung von 1300 Teilen Phosphoroxichlorid und 292 Teilen Phosphorpentachlorid gibt. Das Reaktionsgemisch wird dann 7 Stunden unter Rückfluß gerührt und unter vermindertem Druck eingeengt. Das verbleibende Öl wird in 350 Teilen 1,2-Dichlorethan aufgenommen und über neutrales Aluminiumoxid (Aktivität I) chromatographiert. Nach dem Einengen werden 230 Teile (Ausbeute: 93% d.Th.) 3-Chlor-2-ethyl-5-methyl-2H-1,2,4,6-thiatriazin-1,1-dioxid vom Fp. 57 bis 59°C erhalten. (NMR (60 MHz, $CDCl_3$)

$CH_3$-C 2,3 δ, N-$CH_2$ 3,68 - 4,03δ (q),

$CH_3$-C 1,2 - 1,43 δ(t).

**Beispiel 1**

3,6-Dimethyl-5-[(2'-propyliden)-aminoxy])-2H-1,2,4,6-thiatriazin-1,1-dioxid (Verbindung Nr. 1)

212 Teile 3, 6-Dimethyl-2H-1,2,4,6-thiatriazin(3)on-1,1-dioxid wurden bei Raumtemperatur in eine Mischung aus 292 Teilen Phosphorpentachlorid und 1400 Teilen Phosphoroxichlorid unter Rühren eingeführt und innerhalb 30 Minuten auf 110°C erhitzt. Nach 6 Stunden Rühren unter Rückfluß wurde das Reaktionsgemisch unter vermindertem Druck eingeengt, wobei 234 Teile (Ausbeute: 100 % d. Th.) 5-Chlor-3,6-dimethyl-2H-1,2,4,6-thiatriazin-1,1-dioxid vom Fp. 86 bis 90°C erhalten wurden (NMR 60 MHz, $CDCl_3$):

$CH_3$-C 2,4 δ, $CH_3$-N 3,68 δ).

13,1 Teile Acetonoxim in 30 Teilen Methylenchlorid und 23 Teilen N,N-Dimethylcyclohexylamin in 20 Teilen Methylenchlorid werden über 2 Zuführungen innerhalb von 10 Minuten unter Rühren bei 5 bis 10°C zu einer Lösung von 29,3 Teilen 5-Chlor-3,6-dimethyl-2H-1,2,4,6-thiatriazin-1,1-dioxid in 200 Teilen Methylenchlorid gegeben. Nach einer Stunde Rühren bei Raumtemperatur wird die Reaktionslösung nacheinander mit 1 n Salzsäure, mit 10 %iger Natriumcarbonatlösung gewaschen, über Magnesiumsulfat getrocknet und über Kieselgel chromatographiert. Nach dem Einengen unter vermindertem Druck werden 22 Teile (63 % d. Th.) 3,6-Di-methyl-5-[(2'-propyliden)-aminoxy]-2H-1,2,4,6-thiatriazin-1,1-dioxid vom Fp. 115 bis 118°C erhalten.

**Beispiel 2**

6-Methyl-3-methoxy-5-(2-phenyl-ethoxy)-6H-1,2,4,6-thiatriazin-1,1-dioxid (Verbindung Nr. 2)

22 Teile 2-Phenyl-ethanol und 26,8 Teile Tri-n-propylamin werden bei 0 bis 5°C zu 31,7 Teilen 5-Chlor-6-methyl-3-methoxy-6H-1,2,4,6-thiatriazin-1,1-dioxid in 200 Teilen 1,2-Dichlorethan innerhalb von 10 Minuten unter Rühren gegeben. Nach 15 Minuten Rühren bei Raumtemperatur wird die Reaktionslösung nacheinander mit 1 n Salzsäure, mit 0,5 n Natronlauge und mit Wasser gewaschen, über Magnesiumsulfat getrocknet und über neutralem Aluminiumoxid chromatographiert. Nach dem Einengen unter vermindertem Druck werden 36 Teile (81 % d. Th.) 6-Methyl-3-methoxy-5-(2-phenyl-ethoxy)-6H-1,2,4,6-thiatriazin-1,1-dioxid vom Fp. 74 bis 77°C erhalten.

**Beispiel 3**

6-Methyl-3-methoxy-5-dimethylphosphono-6H-1,2,4,6-thiatriazin-1,1-dioxid (Verbindung Nr. 3)

145 Teile Trimethylphosphit werden bei 38 bis 41°C unter Rühren innerhalb 40 Minuten zu 165 Teilen 5-Chlor-6-methyl-3-methoxy-6H-1,2,4,6-thiatriazin-1,1-dioxid in 550 Teilen Toluol gegeben. Die Reaktionsmischung wird innerhalb 30 Minuten auf 70°C erwärmt und 1 3/4 Stunden bei gleicher Temperatur nachgerührt. Anschließend wird die Lösung von wenigem klebrigen Rückstand abdekantiert und bei 80°C/0,2 mbar von flüchtigen Bestandteilen befreit. Der Rückstand wird in 300 Teilen Essigester gelöst und über neutralem Aluminiumoxid chromatographiert. Nach dem Einengen unter vermindertem Druck werden 192 Teile (96 % d. Th.) 6-Methyl-3-methoxy-5-dimethylphosphono-6H-1,2,4,6-thiatriazin-1,1-dioxid vom

$$n_D^{25} = 1{,}4998 \text{ erhalten.}$$

**Beispiel 4**

6-Methyl-3-methoxy-5-(4-phenyl-n-butoxy)-6H-1, 2, 4, 6-thiatriazin-1,1-dioxid (Verbindung Nr. 4)

Eine Mischung von 27 Teilen 4-Phenyl-n-butanol und 23 Teilen N,N-Dimethyl-cyclohexylamin werden parallel mit 31,7 Teilen 5-Chlor-6-methyl-3-methoxy-6H-1, 2, 4, 6-thiatriazin-1,1-dioxid bei 10 bis 15°C unter Rühren innerhalb 15 Minuten zu 250 Teilen Methylenchlorid gegeben. Nach einer Stunde Rühren bei Raumtemperatur wird das Reaktionsgemisch mit 1 n Salzsäure und mit 10 %iger Natriumcarbonatlösung gewaschen und über Magnesiumsulfat getrocknet. Nach dem Einengen unter vermindertem Druck werden 40 Teile 6-Methyl-3-methoxy-5-(4-phenyl-butoxy)-6H-1,2,4,6-thiatriazin-1,1-dioxid vom Fp. 74 bis 78°C erhalten.

In entsprechender Weise werden die folgenden 1,2,4,6-Thiatriazin-1,1-dioxide der Formel I erhalten:

| Verbindung Nr. | $R^1$ | Y | $R^2$ | $R^3$ | Fp [°C]/$n_D^{25}$ |
|---|---|---|---|---|---|
| 5 | H | O | $CH_2CN$ | H | |
| 6 | H | O | $CH_2CN$ | $CH_3$ | |
| 7 | $CH_3$ | S | $CH_2CN$ | H | |
| 8 | $CH_3$ | O | $CH_2CN$ | $CH_3$ | |
| 9 | $CH_3$ | O | $CH_2CN$ | $C_2H_5$ | |
| 10 | $CH_3$ | O | $CH_2$-$CH_2$-CN | H | |
| 11 | H | O | $CH_2$-$CH_2$-CN | H | |
| 12 | $CH_3$ | O | $CH_2$-$CH_2$-CN | $CH_3$ | 1.5040 |
| 13 | H | O | $CH_2$-$CH_2$-CN | $CH_3$ | |
| 14 | $CH_3$ | S | $CH_2$-$CH_2$-CN | $CH_3$ | |
| 15 | $C_2H_5$ | O | $CH_2$-$CH_2$-CN | $CH_3$ | |
| 16 | $(CH_3)_2N$ | O | $CH_2$-$CH_2$-CN | $CH_3$ | |
| 17 | $CH_3$ | O | $(CH_2)_3$-CN | $CH_3$ | |
| 18 | $CH_3$ | O | $(CH_2)_4$-CN | $CH_3$ | |
| 19 | $CH_3$ | O | $CH_2$-CH=CH-CN | $CH_3$ | |
| 20 | $CH_3$ | O | $CH_2$-C≡C-CN | $CH_3$ | |
| 21 | $CH_3$ | O | $CH_2$-$CH_2$-SCN | i-$C_3H_7$ | |
| 22 | $CH_3$ | O | $(CH_2)_3SCN$ | $CH_3$ | |
| 23 | $CH_3$ | O | $CH_2$-$CH_2$-$NO_2$ | $CH_3$ | |
| 24 | $CH_3$ | O | $(CH_2)_3NO_2$ | $CH_3$ | |
| 25 | $CH_3$ | O | $CH_2$-$CH_2$-O-$CH_2$-CH=$CH_2$ | H | |
| 26 | $(CH_3)_2N$ | O | $CH_2$-$CH_2$-O-$CH_2$-CH=$CH_2$ | $CH_3$ | |
| 27 | $CH_3$ | O | $CH_2$-$CH_2$-O-$CH_2$-CH=$CH_2$ | $CH_3$ | |
| 28 | $CH_3$ | O | $CH_2$-$CH_2$-O-$CH_2$-C≡CH | $CH_3$ | |
| 29 | $CH_3$ | O | $CH_2$-$CH_2$-S-$CH_2$-CH=$CH_2$ | $CH_2$ | |

| Verbindung Nr. | $R^1$ | Y | | $R^2$ | $R^3$ | Fp [°C]/$n_D^{25}$ |
|---|---|---|---|---|---|---|
| 30 | $CH_3$ | O | | $CH_2$-CH=CH-$CH_2$-O-$CH_2$-CH=$CH_2$ | $CH_3$ | |
| 31 | $CH_3$ | O | | $(CH_2)_2$-O-$(CH_2)$-O-$CH_3$ | $CH_3$ | 1,4860 |
| 32 | $CH_3$ | S | | $(CH_2)_2$-O-$(CH_2)_2$-S-$CH_3$ | $CH_3$ | |
| 33 | $CH_3$ | O | | $CH_2$-$CH_2$-$\overset{O}{\overset{\Vert}{C}}$-CH=$CH_2$ | $CH_3$ | |
| 34 | $CH_3$ | O | | $CH_2$-CH=CH-$CO_2CH_3$ | $CH_3$ | |
| 35 | $CH_3$ | O | | $CH_2$-C≡C-$CO_2CH_3$ | $CH_3$ | |
| 36 | $CH_3$ | O | | N=$C(CH_3)_2$ | $CH_3$ | 112-117 |
| 37 | $C_2H_5$ | O | | 2-(4-Chlorphenyl)-ethyl | $CH_3$ | |
| 38 | $CH_3$ | O | | 2-(3-Chlorphenyl)-Ethyl | i-$C_3H_7$ | |
| 39 | $CH_3$ | O | | 2-Phenyl-ethyl | $CH_3$ | 1,5449 |
| 40 | $(CH_3)_2N$ | O | | 2-(2-Chlorphenyl)-ethyl | $CH_3$ | |
| 41 | $CH_3$ | O | | 2-(3-Chlorphenyl)-ethyl | $CH_3$ | |
| 42 | $CH_3$ | O | | 2-(3-Methylphenyl)-ethyl | $CH_3$ | |
| 43 | $CH_3$ | O | | 2-(3-Trifluormethylphenyl)--ethyl | H | |
| 44 | $CH_3$ | O | | 2-(3-Cyanophenyl)-ethyl | $CH_3$ | |
| 45 | $CH_3$ | O | | 2-(3-Methoxyphenyl)-ethyl | $CH_3$ | |
| 46 | $CH_3$ | O | | 3-Phenyl-n-propyl | $CH_3$ | |
| 47 | $CH_3$ | O | | 2-(2,4-Dichlorphenyl)-ethyl | $CH_3$ | |
| 48 | $CH_3$ | O | | 2-(2,4,6-Trichlorphenyl)-ethyl | $CH_3$ | |
| 49 | $CH_3$ | O | | 4-Phenyl-n-butyl | $CH_3$ | |
| 50 | H | O | | 2-Phenyl-ethyl | $CH_3$ | |
| 51 | $CH_3$ | O | | 2-(3-Chlor-4-methoxyphenyl)--ethyl | $CH_3$ | |
| 52 | $CH_3$ | O | | 2-(2-Chlor-4-$CF_3$-phenyl)-ethyl | $CH_3$ | |
| 53 | $(CH_3)_2N$ | O | | Styrol | $CH_3$ | |
| 54 | $CH_3$ | O | | 2-(3-Chlorphenyl)-vinyl | $CH_3$ | |
| 55 | $CH_3$ | O | | 3-Phenyl-prop-2-enyl | $CH_3$ | |
| 56 | $CH_3$ | O | | 3-Phenyl-prop-2-enyl | H | |
| 57 | $CH_3$ | | | $PO(OCH_3)_2$ | $CH_3$ | |
| 58 | $CH_3$ | | | $PO(OC_2H_5)_2$ | $CH_3$ | |
| 59 | $CH_3$ | | | $PO(OCH_3)(OC_2H_5)$ | $CH_3$ | |
| 60 | $CH_3$ | S | | 2-Phenyl-ethyl | $CH_3$ | |
| 61 | $CH_3$ | O | | 2-(4-Chlor-3-methoxy-phenyl)--ethyl | $CH_3$ | |
| 62 | $(CH_3)_2N$ | O | | 2-Phenylethyl | $CH_3$ | |
| 63 | $C_2H_5$ | O | | $CH_2$-$CH_2$-O-$CH_2$-CH=$CH_2$ | $C_2H_5$ | |
| 64 | i-$C_3H_7$ | O | | $CH_2$-$CH_2$-O-$CH_2$-CH=$CH_2$ | $CH_3$ | |
| 65 | n-$C_3H_7$ | S | | $CH_2$-$CH_2$-O-$CH_2$-CH=$CH_2$ | $CH_3$ | |
| 66 | $(CH_3)_2N$ | O | | $CH_2$-$CH_2$-O-$CH_2$-C≡CH | $CH_3$ | |
| 67 | $C_2H_5$ | O | | $CH_2$-$CH_2$-S-$CH_2$-CH=$CH_2$ | $CH_3$ | |
| 68 | $C_2H_5$ | O | | $(CH_2)_2$-O-$(CH_2)_2$-$OCH_3$ | $CH_3$ | |
| 69 | n$C_4H_9$ | O | | $(CH_2)_2$-O-$(CH_2)_2$-O-$CH_3$ | $CH_3$ | |
| 70 | $C_2H_5$ | O | | $(CH_2)_2$O-$(CH_2)_2$-S-$CH_3$ | $CH_3$ | |
| 71 | $C_2H_5$ | O | | N=$C(CH_3)_2$ | $CH_3$ | |
| 72 | $CH_3$ | O | | N=$C(CH_3)_2$ | $C_2H_5$ | |
| 73 | $CH_3$ | O | | 2-Phenylethyl | $C_2H_5$ | 1,5328 |
| 74 | $CH_3$ | O | | 2-(3-Chlorphenyl)-ethyl | $C_2H_5$ | |
| 75 | $CH_3$ | O | | 2-(3-Trifluormethylphenyl)--ethyl | $C_2H_5$ | |
| 76 | $CH_3$ | O | | 2-(3-Methoxyphenyl)-ethyl | i-$C_3H_7$ | |
| 77 | $CH_3$ | O | | 2-(2,4-dichlorphenyl-ethyl | $C_2H_5$ | |
| 78 | $CH_3$ | | | $PO(OCH_3)_2$ | $C_2H_5$ | |
| 79 | $(CH_3)_2N$ | O | | 2-(4-Cyanophenyl)-ethyl | $C_2H_5$ | |
| 80 | $C_2H_5$ | O | | 2-Phenylethyl | $CH_2$-$CH_2$-Cl | |
| 81 | $CH_3$ | O | | $CH_2$-$CH_2$-CN | $CH_2$-$CH_2$-Cl | |
| 82 | $CH_3$ | O | O | $CH_2CN$ | H | |
| 83 | $CH_3$ | S | O | $CH_2CN$ | $CH_3$ | |
| 84 | $CH_3$ | O | S | $CH_2CN$ | H | |

| Verbin-dung Nr. | $R^4$ ($R^1 = R^4$-X-) | X | Y | $R^2$ | $R^3$ | Fp [°C]/$n_D^{25}$ |
|---|---|---|---|---|---|---|
| 85 | $CH_3$ | O | O | $CH_2CN$ | $CH_3$ | |
| 86 | $CH_3$ | O | O | $CH_2CN$ | $C_2H_5$ | |
| 87 | $CH_3$ | O | O | $CH_2-CH_2-CN$ | H | |
| 88 | $CH_3$ | O | O | $CH_2-CH_2-CN$ | $CH_3$ | 162-165 |
| 89 | $CH_3$ | O | S | $CH_2-CH_2-CN$ | $CH_3$ | 129-132 |
| 90 | $CH_3$ | O | O | $(CH_2)_3-CN$ | H | |
| 91 | $CH_3$ | O | O | $(CH_2)_3-CN$ | $CH_3$ | |
| 92 | $CH_3$ | O | O | $(CH_2)_3-CN$ | $CH_3$ | |
| 93 | $CH_3$ | O | O | $CH_2-CH_2-CN$ | $i-C_3H_7$ | |
| 94 | $CH_3$ | O | O | $(CH_2)_3-CN$ | $CH_3$ | |
| 95 | $CH_3$ | O | O | $(CH_2)_4-CN$ | $CH_3$ | |
| 96 | $CH_3$ | O | O | $CH_2-CH=CH-CN$ | $CH_3$ | |
| 97 | $CH_3$ | O | O | $CH_2-C\equiv C-CN$ | $CH_3$ | |
| 98 | $CH_3$ | O | O | $CH_2-CH_2-SCN$ | $CH_3$ | |
| 99 | $CH_3$ | O | O | $(CH_2)_3SCN$ | $CH_3$ | |
| 100 | $CH_3$ | O | O | $CH_2-CH_2-NO_2$ | $CH_3$ | |
| 101 | $CH_3$ | O | O | $(CH_2)_3NO_2$ | $CH_3$ | |
| 102 | $CH_3$ | O | O | $CH_2-CH_2-O-CH_2-CH=CH_2$ | H | |
| 103 | $CH_3$ | S | O | $CH_2-CH_2-O-CH_2-CH=CH_2$ | $CH_3$ | |
| 104 | $CH_3$ | O | O | $CH_2-CH_2-O-CH_2-CH=CH_2$ | $CH_3$ | 1.5049 |
| 105 | $CH_3$ | O | O | $CH_2-CH_2-O-CH_2-C=CH$ | $CH_3$ | 1.5009 |
| 106 | $CH_3$ | O | O | $CH_2-CH_2-S-CH_2-CH=CH_2$ | $CH_3$ | |
| 107 | $CH_3$ | O | O | $CH_2-CH=CH-CH_2-O-CH_2-CH=CH_2$ | $CH_3$ | |
| 108 | $CH_3$ | O | O | $(CH_2)_2-O-(CH_2)_2-O-CH_3$ | $CH_3$ | 36-39 |
| 109 | $CH_3$ | O | S | $(CH_2)_2-O-(CH_2)_2-S-CH_3$ | $CH_3$ | |
| 110 | $CH_3$ | O | O | $CH_2-CH_2-\overset{O}{\overset{\|}{C}}-CH=CH_2$ | $CH_3$ | |
| 111 | $CH_3$ | O | O | $CH_2-CH=CH-CO_2CH_3$ | $CH_3$ | |
| 112 | $CH_3$ | O | O | $CH_2-C\equiv C-CO_2CH_3$ | $CH_3$ | |
| 113 | $CH_3$ | O | O | $N=C(CH_3)_2$ | $CH_2-CH_2-Cl$ | |
| 114 | $C_2H_5$ | O | O | 2-(4-Chlorphenyl)-ethyl | $CH^3$ | |
| 115 | $CH_3$ | O | O | 2-(3-Chlorphenyl)-ethyl | $CH_3$ | |
| 116 | $CH_3$ | O | O | 2-Phenylethyl | $i-C_3H_7$ | |
| 117 | $CH_3$ | O | O | 2-(2-Chlorphenyl)-ethyl | $CH_3$ | |
| 118 | $CH_3$ | O | O | 2-(3-Chlorphenyl)-ethyl | $CH_3$ | 93-95 |
| 119 | $CH_3$ | O | O | 2-(3-Methylphenyl)-ethyl | $CH_3$ | |
| 120 | $CH_3$ | S | O | 2-(3-Trifluormethylphenyl)--ethyl | H | |
| 121 | $CH_3$ | O | O | 2-(3-Cyanophenyl)-ethyl | $CH_3$ | |
| 122 | $CH_3$ | O | O | 2-(3-Methoxyphenyl)-ethyl | $CH_3$ | |
| 123 | $CH_3$ | O | O | 3-Phenyl-n-propyl | $CH_3$ | 1.5430 |
| 124 | $CH_3$ | O | O | 2-(3,4-Dichlorphenyl)-ethyl | $CH_3$ | |
| 125 | $CH_3$ | O | O | 2-(2,4,6-Trichlorphenyl)-ethyl | $CH_3$ | |
| 126 | $CH_3$ | S | O | 4-Phenyl-n-butyl | $CH_3$ | |
| 127 | $CH_3$ | O | O | 2-Phenyl-ethyl | H | |
| 128 | $CH_3$ | O | O | 2-(3-Chlor-4-methoxyphenyl)-ethyl | $CH_3$ | |
| 129 | $CH_3$ | O | O | 2-(2-Chlor-4-trifluormethyl-phenyl)-ethyl | $CH_3$ | |
| 130 | $CH_3$ | O | O | Styryl | $CH_3$ | |
| 131 | $CH_3$ | O | O | 2-(3-Chlorphenyl)-vinyl | $CH_3$ | |
| 132 | $CH_3$ | O | O | 3-Phenyl-prop-2-enyl | $CH_3$ | |
| 133 | $CH_3$ | O | O | 3-Phenyl-prop-2-enyl | H | |
| 134 | $CH_3$ | O | | $PO(OC_2H_5)_2$ | $CH_3$ | 84-86 |
| 135 | $CH_3$ | O | | $PO(OCH_3)_2$ | H | |
| 136 | $CH_3$ | S | | $PO(OCH_3)(OC_2H_5)$ | $CH_3$ | |
| 137 | $CH_3$ | O | S | 2-Phenyl-ethyl | $CH_3$ | |
| 138 | $CH_3$ | O | O | 2-(4-Chlor-3-methoxy-phenyl)--ethyl | $CH_3$ | |

| Verbin-dung Nr. | $R^4$ ($R^1 = R^4$-X-) | X | Y | $R^2$ | $R^3$ | Fp [°C]/$n_D^{25}$ |
|---|---|---|---|---|---|---|
| 139 | $CH_3$ | S | O | 2-Phenyl-ethyl | $CH_3$ | |
| 140 | $C_2H_5$ | O | O | $CH_2CH_2$-O-$CH_2$-CH=$CH_2$ | $CH_3$ | |
| 141 | i-$C_3H_7$ | O | O | $CH_2$-$CH_2$-O-$CH_2$-CH=$CH_2$ | $CH_3$ | |
| 142 | n-$C_3H_7$ | O | S | $CH_2$-$CH_2$-O-$CH_2$-CH=$CH_2$ | $CH_3$ | |
| 143 | $CH_3$ | O | O | $CH_2$-$CH_2$-O-$CH_2$-C≡CH | $C_2H_5$ | |
| 144 | $C_2H_5$ | O | O | $CH_2$-$CH_2$-S-$CH_2$-CH=$CH_2$ | $CH_3$ | |
| 145 | $C_2H_5$ | O | O | $(CH_2)_2$-O-$(CH_2)_2$-$OCH_3$ | $CH_3$ | |
| 146 | n-$C_4H_9$ | O | O | $(CH_2)_2$-O-$(CH_2)_2$-O-$CH_3$ | $CH_3$ | |
| 147 | $C_2H_5$ | O | O | $(CH_2)_2$-O-$(CH_2)_2$-S-$CH_3$ | $CH_3$ | |
| 148 | $CH_3$ | O | O | N=$C(CH_3)_2$ | $CH_3$ | 132-135 |
| 149 | $CH_3$ | S | O | N=$C(CH_3)_2$ | $C_2H_5$ | |
| 150 | $CH_3$ | O | O | 2-Phenylethyl | $C_2H_5$ | |
| 151 | $CH_3$ | O | O | 2-(3-Chlorphenyl)-ethyl | $C_2H_5$ | |
| 152 | $CH_3$ | S | O | 2-(3-Trifluormethylphenyl)--ethyl | $C_2H_5$ | |
| 153 | $CH_3$ | O | O | 2-(3-Methoxyphenyl)-ethyl | i-$C_3H_7$ | |
| 154 | $CH_3$ | O | O | 2-(2,4-Dichlorpehnyl)-ethyl | $C_2H_5$ | |
| 155 | $CH_3$ | O | | $PO(OCH_3)_2$ | $C_2H_5$ | |
| 156 | $CH_3$ | O | S | 2-(4-Cyanophenyl)-ethyl | $C_2H_5$ | |
| 157 | $C_2H_5$ | O | O | 2-Phenylethyl | $CH_2$-$CH_2$-Cl | |
| 158 | $CH_3$ | O | O | 2-Cyanoethyl | $CH_2$-$CH_2$-Cl | |
| 159 | $CH_3$ | O | O | $CH_2$-CH[CN]$CH_3$ | $CH_3$ | |
| 160 | $CH_3$ | O | O | $(CH_2)_4$SCN | $CH_3$ | |
| 161 | $CH_3$ | O | O | $(CH_2)_2$-O$(CH_2)_2$-allyl | $CH_3$ | |
| 162 | $CH_3$ | O | O | $CH_2$CH≡CH-$CO_2H_5$ | $CH_3$ | |
| 163 | $C_2H_5$ | O | S | $CH_2$-C≡C-$CO_2C_2H_5$ | $CH_3$ | |
| 164 | $CH_3$ | O | O | N=$C(CH_3)C_2H_5$ | $CH_3$ | |
| 165 | $CH_3$ | O | O | N=CH-$CH_3$ | $CH_3$ | |
| 166 | $CH_3$ | O | O | N=CH-$C_2H_5$ | $CH_3$ | |
| 167 | $CH_3$ | O | O | 2-(4-Chlorphenyl)-ethyl | $CH_3$ | 117-121 |
| 168 | $CH_3$ | O | O | 2-(3,4-Dichlorphenyl)-ethyl | $CH_3$ | |
| 169 | $CH_3$ | O | O | 2-(4-Cyanophenyl)-ethyl | $CH_3$ | |
| 170 | $CH_3$ | S | O | 2-(3-Trifluormethoxyphenyl)--ethyl | $CH_3$ | |
| 171 | $CH_3$ | O | O | 2-Nitrobenzyl | $CH_3$ | 171-173 |
| 172 | $CH_3$ | O | O | 2-Chlor-4-nitrobenzyl | $CH_3$ | |
| 173 | $CH_3$ | O | S | 4-Nitrobenzyl | $CH_3$ | |
| 174 | $CH_3$ | O | O | 4-Nitrobenzyl | $CH_3$ | 187-190 |
| 175 | $CH_3$ | O | O | 3-Nitrobenzyl | $CH_3$ | 171-174 |
| 176 | $CH_3$ | O | O | 2-Chlor-6-nitrobenzyl | $CH_3$ | 163-166 |
| 177 | $CH_3$ | O | O | 2-(2-Nitrophenoxy)-ethyl | $CH_3$ | |
| 178 | $CH_3$ | O | O | 2-(3-Nitrophenoxy)-ethyl | $CH_3$ | |
| 179 | $CH_3$ | O | O | 2-(4-Cyanophenoxy)-ethyl | $CH_3$ | |
| 180 | $CH_3$ | O | S | 2-(3-Trifluormithylphenoxy)-ethyl | $CH_3$ | |
| 181 | $CH_3$ | O | O | 2-(3-Trifluormethoxyphenoxy)--ethyl | $CH_3$ | |
| 182 | $CH_3$ | O | O | 2-(4-Chlorphenoxy)-ethyl | $CH_3$ | 136-138 |
| 183 | $CH_3$ | O | O | 2-(3-Chlorphenoxy)-ethyl | $CH_3$ | 88-90 |
| 184 | $CH_3$ | O | O | 2-(2-Chlorphenoxy)-ethyl | $CH_3$ | |
| 185 | $CH_3$ | O | O | 2-(3,5-Dichlorphenoxy)-ethyl | $CH_3$ | |
| 186 | $CH_3$ | O | O | 2-(2,4-Dichlorphenoxy)ethyl | $CH_3$ | 127-129 |
| 187 | $CH_3$ | O | O | 2-(2,4,6-Trichlorphenoxy)--ethyl | $CH_3$ | |
| 188 | $CH_3$ | O | O | 2-(2-Chlor-4-methylphenoxy)--ethyl | $CH_3$ | |
| 189 | $CH_3$ | O | O | 2-(2,4-Dimethyl-phenoxy)--ethyl | $CH_3$ | |
| 190 | $CH_3$ | O | O | 2-(2-Chlor-4-trifluormethyl--phenoxy)-ethyl | $CH_3$ | |
| 191 | $CH_3$ | O | O | 2-(3-Methoxy-phenoxy)-ethyl | $CH_3$ | |
| 192 | $CH_3$ | O | O | 2-Phenoxy-vinyl | $CH_3$ | |

| Verbin-dung Nr. | $R^4$ ($R^1 = R^4$-X-) | X | Y | $R^2$ | $R^3$ | Fp [°C]/$n_D^{25}$ |
|---|---|---|---|---|---|---|
| 193 | $CH_3$ | O | O | 3-Phenoxy-prop-1-enyl | $CH_3$ | |
| 194 | $CH_3$ | S | O | 2-(4-Chlorphenoxy)-ethyl | $CH_3$ | |
| 195 | $CH_3$ | O | O | 4-Phenoxy-n-but-2-inyl | $CH_3$ | 98-101 |
| 196 | $CH_3$ | O | O | 4-(4-Chlorphenoxy)-n-but-2--inyl | $CH_3$ | |
| 197 | $CH_3$ | O | O | 4-(2,4-Dichlorphenoxy)-n-but--2-inyl | $CH_3$ | |
| 198 | $CH_3$ | O | O | 4-(3-Chlorphenoxy)-n-but-2--inyl | $CH_3$ | |
| 199 | $CH_3$ | O | O | 4-(4-Fluorphenoxy)-n-but-2--inyl | $CH_3$ | 102-104 |
| 200 | $CH_3$ | O | O | 4-(2,4-Dichlorphenoxy)-n--but-2-inyl | $CH_3$ | |
| 201 | $CH_3$ | O | O | 4-(2-Methyl-4-Chlorphenoxy)--n-but-2-inyl | $CH_3$ | |
| 202 | $CH_3$ | O | O | $CH(CH=CH_2)-CO_2C_2H_5$ | $CH_3$ | 1.5018 |
| 203 | $CH_3$ | O | O | 2-Phenoxy-ethyl | $CH_3$ | 81-84 |
| 204 | $CH_3$ | O | O | 2-(2-Methylphenyl)-ethyl | $CH_3$ | 102-104 |
| 205 | $CH_3$ | O | O | 2-Phenyl-n-propyl | $CH_3$ | 67-68 |
| 206 | $CH_3$ | O | O | 3-(2,4-Dichlorphenoxy)--n-propyl | $CH_3$ | 96-98 |
| 207 | $CH_3$ | O | O | 3-(4-Chlorphenoxy)-n-propyl | $CH_3$ | 120-123 |
| 208 | $CH_3$ | O | O | 3-(3-Trifluormethylphenoxy)--n-propyl | $CH_3$ | 70-73 |
| 209 | $CH_3$ | O | O | 3-(4-Chlorphenyl)-n-propyl | $CH_3$ | |
| 210 | $CH_3$ | O | O | 3-(2,4-Dichlorphenyl)-n--propyl | $CH_3$ | |
| 211 | $CH_3$ | O | O | 3-(3-Chlorphenoxy)-n-propyl | $CH_3$ | 125-128 |
| 212 | $CH_3$ | O | S | 2-(3-Chlorphenyl)-ethyl | $CH_3$ | 67-69 |
| 213 | $CH_3$ | O | O | 3-(p-Tolyl)-n-propyl | $CH_3$ | |
| 214 | $CH_3$ | O | O | 2-(2-Chlorphenyl)-ethyl | $CH_3$ | 116-119 |
| 215 | $CH_3$ | O | O | 2-(4-Methyl-2-methoxy-phenoxy)-ethyl | $CH_3$ | 96-99 |
| 216 | $C_2H_5$ | O | O | $(CH_2)_2-O-(CH_2)_2-O-CH_3$ | $C_2H_5$ | 1,4879 |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Fasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Fasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem

Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90-Gewichtsprozent, Wirkstoff.

**Beispiele für Formulierungen sind:**

I. Man vermischt 90 Gewichtsteile der Verbindung gemäß Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 175 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 174 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 183 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 182 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 176 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 171 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen oder während des Auflaufens der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 10 kg/ha und

mehr, vorzugsweise 0,5 bis 4,0 kg/ha, wobei sich die höheren Dosen besonders zur totalen Bekämpfung von Vegetationen eignen.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Bei Soja und Reis wird zusätzlich etwas Torfmull beigemischt, um ein einwandfreies Wachstum zu gewährleisten. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Die Aufwandmengen variieren je nach Verbindung; sie betragen 0,5, 1,0, 2,0 oder 4,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird. Zum Zwecke der Nachauflaufbehandlung zieht man die Testpflanzen je nach Wuchsform erst bis zu einer Wuchshöhe von 3 bis 10 cm an und behandelt sie danach.

Die Versuchsgefäße werden im Gewächshaus aufgestellt, wobei für wärmeliebende Arten wärmere Bereiche (20 bis 30°C) und für solche gemäßigter Klimate 15 bis 25°C bevorzugt werden. Die Versuchsperiode erstreckt sich über 3 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Testpflanzen handelt es sich um Datura stramonium (gemeiner Stechapfel), Galium aparine (Klettenlabkraut), Oryza sativa (Reis), Sinapis alba (Weißer Senf), Solanum nigrum (schwarzer Nachtschatten), Gossypium hirsutum (Baumwolle), Glycine max. (Sojabohnen), Triticum aestivum (Weizen).

Die Gewächshausversuche zeigen, daß die neuen Verbindungen bei Vor- und Nachauflaufanwendung zur Bekämpfung von unerwünschten Pflanzen geeignet sind. Von besonderem Interesse ist dabei ihre Verwendung als selektive herbizide Mittel in Kulturpflanzenbeständen.

Bei Vorauflaufanwendung bekämpfen die Wirkstoffe Nr 171, 174, 175, 176, 182 und 183 die beispielhaft eingesetzten unerwünschten Pflanzen, wobei die Kulturpflanzen nicht oder nur gering geschädigt werden. Ferner zeigen die Verbindungen Nr. 176 und Nr. 183 bei Nachauflaufwendung von 2,0 kg Wirkstoff/ha eine gute Wirkung gegen unerwünschte Pflanzen.

Die Mittel können angewendet werden, wenn sich sowohl Kulturpflanzen als auch unerwünschte Pflanzen gleichzeitig im Vorauflauf- oder Nachauflaufstadium befinden. Die Anwendung kann aber erfolgen, wenn die Kulturpflanzen zwar schon etabliert sind, die Unkräuter und Ungräser aber noch nicht aufgelaufen sind oder diese sich gerade im Auflaufen befinden. Sind gewisse, bereits stehende Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die unbedeckte Bodenfläche gelangen und die darin keimenden und aufwachsenden unerwünschten Pflanzen erreichen (post-directed, lay-by, Unterblattspritzung.

In Betracht kommen beispielsweise folgende Kulturen:

| **Botanischer Name** | **Deutscher Name** |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |

| Botanischer Name | Dutscher Name |
|---|---|
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum | Baumwolle |
| (Gossypium arboreum | |
| Gossypium herbaceum | |
| Gossypium vitifolium) | |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum | Tabak |
| (N. rustica) | |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche**

1. 1, 2, 4, 6-Thiatriazin-1,1-dioxide der Formel

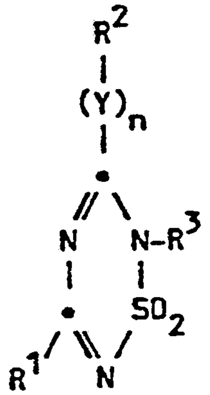

(I),

in der

$R_1$ Wasserstoff, einen unverzweigten oder verzweigten Alkyl- oder Alkenylrest mit bis zu 10 Kohlenstoffatomen, Propargyl, But-1-in-3-yl, But-2-inyl, einen durch Halogen substituierten gesättigten, unverzweigten oder verzweigten aliphatischen Rest mit bis zu 10 Kohlenstoffatomen, 2-Methoxyethyl, 2-Ethoxyethyl, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Methoxy-n-butyl, 4-Methoxy-n-butyl, 2-Methylmercapto-ethyl, 2-Ethylmercapto-ethyl, 3-Methylmercapto-n-Aropyl, 3-Methylmercapto-n-butyl, 1-Methylmercapto-sec.-butyl, Methylmercapto-tert.butyl, 2-Methylmercapto-n-butyl, einen Alkylamino- oder Dialkylaminorest mit 1 bis 6 Kohlenstoffatomen in einer Alkylgruppe, einen Cycloalkylrest mit 3 bis 7 Kohlenstoffatomen, einen gegebenenfalls durch Halogen, Alkyl oder Alkoxy mit 1 bis 4 Kohlenstoffatomen substituierten Phenylrest, einen gegebenenfalls durch Halogen substituierten Benzylrest oder den Rest $R^4$-X-, wobei $R^4$ die für $R^1$ genannten Bedeutungen ausgenommen Alkylamino- und Dialkylamino, hat und X Sauerstoff Schwefel, -SO- oder -$SO_2$-bedeutet,

$R^2$ einen durch Cyano, Thiocyano, Nitro oder Vinylketo substituierten gesättigten oder ungesättigten, unverzweigten oder verzweigten aliphatischen Rest mit bis zu 10 Kohlenstoffatomen, 2-Allyloxyethyl, 2-Propargyloxyethyl, 2-Allyloxy-n-Aropyl, 2-Propargyloxy-n-propyl, 3-Allyloxy-n-profyl, 4-Allyloxy-but-2-inyl, 2-Allylmercaptoethyl, 2-Propargylmercaptoethyl, 2-Allylmercapto-n-propyl, einen durch Alkyl, Alkenyl oder Alkinyl mit jeweils bis zu 4 Kohlenstoffatomen substituierten Alkylenglykolalkyl-, -alkenyl- oder -alkinylrest mit bis zu 6 Kohlenstoffatomen, einen durch Alkoxycarbonyl oder Alkylmercaptocarbonyl mit bis zu 5 Kohlenstoffatomen substituierten Alkenyl- oder Alkinylrest mit jeweils bis zu 4 Kohlenstoffatomen, einen Alkylidenamino- oder Dialkylmethylidenaminorest mit bis zu 6 Kohlenstoffatomen, einen gegebenenfalls durch Halogen, Alkyl, Halogenalkyl, Alkoxy, Halogenalkoxy, Alkylmercapto oder Halogenalkylmercapto mit jeweils bis zu 4 Kohlenstoffatomen, Nitro, Cyano, Thiocyano am Phenylring substituierten Phenylalkyl-, Phenylalkenyl- oder Phenylalkinylrest mit 8 bis 12 Kohlenstoffatomen,

$R_3$ Wasserstoff, einen unverzweigten oder versweigten Alkyl- oder Alkenylrest mit bis zu 10 Kohlenstoffatomen, Propargyl. But-1-in-3-yl, But-2-inyl, einen durch Halogen substituierten gesättigten, unverzweigten oder verzweigten aliphatischen Rest mit bis zu 10 Kohlenstoffatomen, 2-Methoxyethyl, 2-Ethoxyethyl, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Methoxy-n-butyl, 4-Methoxy-n-butyl, oder einen Cycloalkylrest mit 3 bis 7 Kohlenstoffatomen und

Y Sauerstoff, Schwefel, -SO- oder -$SO_2$- und

n 1 bedeuten,

oder in der $R^2$ einen durch Nitro oder Halogen und Nitro substituierten Benzylrest oder einen durch Halogen, Alkyl, Halogenalkyl, Alkoxy, Halogenalkoxy, Alkylmercapto oder Halogenalkylmercapto mit jeweils bis zu 4 Kohlenstoffatomen, Nitro, Cyano, Thiocyano am Phenylring substituierten Phenoxyalkyl-, Phenoxyalkenyl- oder Phenoxyalkinylrest mit 8 bis 12 Kohlenstoffatomen bedeutet, wenn $R^1$ den Rest $R^4$-X- und n 1 bedeuten, oder in der $R_2$ einen Dialkylphosphonorest bedeutet, wenn n 0 ist.

2. 1,2,4,6-Thiatriazin-1,1-dioxid der Formel I gemäß Anspruch 1, dadurch gekennzeichnet, daß $R^1$ für den Rest $R^4$-X-, wobei $R^4$ Alkyl mit 1 bis 4 Kohlenstoffatomen und X Sauerstoff bedeuten, $R^2$ für einen Dialkylphosphonorest mit jeweils 1 bis 4 Kohlenstoffatomen in einer Alkylgruppe und $R^3$ für Alkyl mit 1 bis 4 Kohlenstoffatomen stehen und n 0 bedeutet.

3. 1,2,4-Thiatriazin-1,1-dioxide der Formel I gemäß Anspruch 1, dadurch gekennzeichnet, daß $R^1$ den Rest $R^4$-X- bedeutet wobei $R^4$ Alkyl mit 1 bis 4 Kohlenstoffatomen und X Sauerstoff oder Schwefel bedeuten, und $R^2$ für einen gegebenenfalls durch Halogen am Phenylring substituierten Phenoxyalkylrest mit 7 bis 10 Kohlenstoffatomen, $R^3$ für Alkyl mit 1 bis 4 Kohlenstoffatomen und Y für Sauerstoff oder Schwefel stehen.

4. 1,2,4,6-Thiatriazin-1,1-dioxide der Formel I gemäß Anspruch 1, dadurch gekennzeichnet, daß $R^1$ und $R^3$ für Alkyl mit 1 bis 4 Kohlenstoffatomen, $R^2$ für einen durch Cyano substituierten, gesättigten oder ungesättigten aliphatischen Rest mit bis zu 4 Kohlenstoffatomen und Y für Sauerstoff oder Schwefel stehen.

5. 1,2,4,6-Thiatriazin-1,1-dioxide der Formel I gemäß Anspruch 1, dadurch gekennzeichnet, daß $R^1$ den Rest $R^4$-X- bedeutet, wobei $R^4$ Alkyl mit 1 bis 4 Kohlenstoffatomen und X Sauerstoff oder Schwefel bedeuten, und $R^2$ für einen gegebenenfalls durch Halogen, Cyano, Nitro oder Alkyl am Phenylring substituierten Phenylalkyl-, Phenylalkenyl- oder Phenylalkinylrest mit 8 bis 12 Kohlenstoffatomen, $R^3$ für Alkyl mit 1 bis 4 Kohlenstoffatomen und Y für Sauerstoff oder Schwefel stehen.

6. Verfahren zur Herstellung von 2H-1,2,4,6-Thiatriazin-1,1-dioxiden der Formel I gemäß Anspruch 1, dadurch gekennzeichnet, daß man ein substituiertes 1,2,4,6-Thiatriazin-1,1-dioxid der Formel

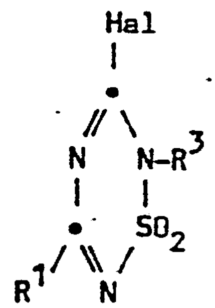
(II),

in der $R^1$ und $R^3$ die obengenannten Bedeutungen baben und Hal für Halogen steht, mit einer Verbindung der Formel

$$H - (Y)_n - R^2 \qquad (III),$$

in der $R^2$, Y und n die obengenannten Bedeudeutungen haben oder einem Alkali-, Erdalkali- oder Ammoniumsalz einer Verbindung der Formel III, gegebenenfalls in einem inerten organischen Lösungsmittel und gegebenenfalls in Anwesenheit eines Säureakzeptors umsetzt.

7. Herbizid, enthaltend ein 1,2,4,6-Thiatriazin-1,1-dioxid der Formel I gemäß Anspruch 1.

8. Herbizid, enthaltend inerte Zusatzstoffe und ein 1,2,4,6-Thiatriazin-1,1-dioxid der Formel I gemäß Anspruch 1.

9. Herbizid, enthaltend inerte Zusatzstoffe und ein 1,2,4,6-Thiatriazin-1,1-dioxid der Formel I gemäß Anspruch 1, worin $R^1$ den Rest $R^4$-X- bedeutet, wobei $R^4$ Alkyl mit 1 bis 4 Kohlenstoffatomen und X Sauerstoff oder Schwefel bedeuten, und $R^2$ für einen gegebenenfalls durch Halogen, Cyano, Nitro oder Alkyl am Phenylring substituierten Phenylalkyl-, Phenylalkenyl- oder Phenylalkinylrest mit 8 bis 12 Kohlenstoffatomen, $R^3$ für Alkyl mit 1 bis 4 Kohlenstoffatomen und Y für Sauerstoff oder Schwefel stehen.

10. Verfahren zur Bekämpfung unerwünschten Pflanzenwuchses, dadurch gekennzeichnet, daß man die Pflanzen und/oder den Boden mit einer Herbizid wirksamen Menge eines 2H-1,2,4,6-Thiatriazin-1,1-dioxids der Formel I gemäß Anspruch 1 behandelt.

**Claims**

1. A 1,2,4,6-thiatriazine-1,1-dioxide of the formula

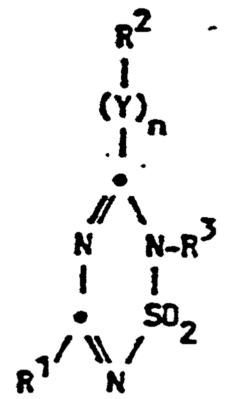

(I),

where

$R^1$ is hydrogen, a straight-chain or branched alkyl or alkenyl of up to 10 carbon atoms, propargyl, but-1-yn-3-yl, but-2-ynyl or a saturated, straight-chain or branched aliphatic radical of up to 10 carbon atoms which is substituted by halogen, or is 2-methoxyethyl, 2-ethoxyethyl, 3-methoxy-n-propyl, 2-methoxyisopropyl, 3-methoxy-n-butyl, 1-methoxy-sec.-butyl, methoxy-tert.-butyl, ethoxy-tert.-butyl, 2-methoxy-n-butyl, 4-methoxy-n-butyl, 2-methyl-mercaptoethyl, 2-ethylmercaptoethyl, 3-methylmercapto-n-propyl, 3-methylmercapto-n-butyl, 1-methylmercapto-sec.-butyl, methylmercapto-tert.-butyl or 2-methylmercapto-n-butyl, or is an alkylamino or dialkylamino radical where alkyl is of 1 to 6 carbon atoms, cycloalkyl of 3 to 7 carbon atoms, or phenyl which is unsubstituted or substituted by halogen, alkyl or alkoxy of 1 to 4 carbon atoms, or is unsubstituted or halogen-substituted benzyl, or is $R^4$-X-, where $R^4$ has the meanings given for $R^1$, except alkylamino and dialkylamino, and X is oxygen, sulfur, -SO- or $SO_2$-,

$R^2$ is a saturated or unsaturated, straight-chain or branched aliphatic radical of up to 10 carbon atoms which is substituted by cyano, thiocyano, nitro or vinylketo, or is 2-allyloxyethyl, 2-propargyloxyethyl, 2-allyloxy-n-propyl, 2-propargyloxy-n-propyl, 3-allyloxy-n-propyl, 4-allyloxy-but-2-ynyl, 2-allylmercaptoethyl, 2-propargylmercaptoethyl or 2-allylmercapto-n-propyl, or is an alkyleneglycolalkyl, alkyleneglycolalkenyl or alkyleneglycolalkynyl radical of up to 6 carbon atoms which is substituted by alkyl, alkenyl or alkynyl, each having up to 4 carbon atoms, or is an alkenyl or alkynyl radical, each having up to 4 carbon atoms, which is substituted by alkoxycarbonyl or alkylmercaptocarbonyl of up to 5 carbon atoms, or is an alkylideneamino or dialkylmethylideneamino radical of up to 6 carbon atoms, or a phenylalkyl, phenylalkenyl or phenylalkynyl radical of 8 to 12 carbon atoms which is unsubstituted or substituted in the phenyl ring by halogen, alkyl, haloalkyl, alkoxy, haloalkoxy, alkylmercapto or haloalkylmercapto, each having up to 4 carbon atoms, nitro, cyano or thiocyano,

$R^3$ is hydrogen, a straight-chain or branched alkyl or alkenyl of up to 10 carbon atoms, propargyl, but-1-yn-3-yl, but-2-ynyl or a saturated, straight-chain or branched aliphatic radical of up to 10 carbon atoms which is substituted by halogen, or is 2-methoxyethyl, 2-ethoxyethyl, 3-methoxy-n-propyl, 2-methoxyisopropyl, 3-methoxy-n-butyl, 1-methoxy-sec.-butyl, methoxy-tert.-butyl, ethoxy-tert.-butyl, 2-methoxy-n-butyl or 4-methoxy-n-butyl, or is cycloalkyl of 3 to 7 carbon atoms,

Y is oxygen, sulfur, -SO- or $-SO_2$-, and

n is 1,

or where $R^2$ is benzyl which is substituted by nitro or by halogen and nitro, or is a phenoxyalkyl, phenoxyalkenyl or phenoxyalkynyl radical of 8 to 12 carbon atoms which is substituted in the phenyl ring by halogen, alkyl, haloalkyl, alkoxy, haloalkoxy, alkylmercapto or haloalkylmercapto, each having up to 4 carbon atoms, nitro, cyano or thiocyano, when $R^1$ is $R^4$-X- and n is 1,

or where $R^2$ is dialkylphosphono when n is 0.

2. A 1,2,4,6-thiatriazine-1,1-dioxide of the formula I as claimed in claim 1, where $R^1$ is $R^4$-X-, $R^4$ denoting alkyl of 1 to 4 carbon atoms and X denoting oxygen, $R^2$ is dialkylphosphono, where alkyl is of 1 to 4 carbon atoms, $R^3$ is alkyl of 1 to 4 carbon atoms, and n is 0.

3. A 1,2,4,6-thiatriazine-1,1-dioxide of the formula I as claimed in claim 1, where $R^1$ is $R^4$-X-, $R^4$ denoting alkyl of 1 to 4 carbon atoms and X denoting oxygen or sulfur, $R^2$ is phenoxyalkyl of 7 to 10 carbon atoms which is unsubstituted or substitued in the phenyl ring by halogen, $R^3$ is alkyl of 1 to 4 carbon atoms, and Y is oxygen or sulfur.

4. A 1,2,4,6-thiatriazine-1,1-dioxide of the formula I as claimed in claim 1, where $R^1$ and $R^3$ are each alkyl of 1 to 4 carbon atoms, $R^2$ is a cyano-substituted, saturated or unsaturated aliphatic radical of up to 4 carbon atoms, and Y is oxygen or sulfur.

5. A 1,2,4,6-thiatriazine-1,1-dioxide of the formula I as claimed in claim 1, where $R^1$ is $R^4$-X-, $R^4$ denoting alkyl of 1 to 4 carbon atoms and X denoting oxygen or sulfur, $R^2$ is phenylalkyl, phenylalkenyl or phenylalkynyl of 8 to 12 carbon atoms which is unsubstituted or substituted in the phenyl ring by halogen, cyano, nitro or alkyl, $R^3$ is

alkyl of 1 to 4 carbon atoms, and Y is oxygen or sulfur.

6. A process for the preparation of a 2H-1,2,4,6-thiatriazine-1,1-dioxide of the formula I as claimed in claim 1, wherein a substituted 1,2,4,6-thiatriazine-1,1-dioxide of the formula

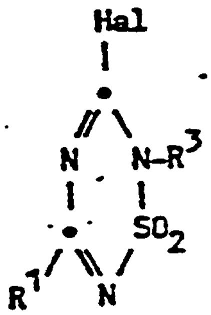

(II),

where $R^1$ and $R^3$ have the above meanings and Hal is halogen, is reacted with a compound of the formula

$$H\text{-}(Y)_n\text{-}R^2 \qquad (III),$$

where $R^2$, Y and n have the above meanings, or with an alkali metal salt, alkaline earth metal salt or ammonium salt of a compound of the formula III, in the presence or absence of an inert organic solvent, and in the presence or absence of an acid acceptor.

7. A herbicide containing a 1,2,4,6-thiatriazine-1,1-dioxide of the formula I as claimed in claim 1.

8. A herbicide containing inert additives and a 1,2,4,6-thiatriazine-1,1-dioxide of the formula I as claimed in claim 1.

9. A herbicide containing inert additives and a 1,2,4,6-thiatriazine-1,1-dioxide of the formula I as claimed in claim 1, where $R^1$ is $R^4$-X-, $R^4$ denoting alkyl of 1 to 4 carbon atoms and X denoting oxygen or sulfur, $R^2$ is phenylalkyl, phenylalkenyl or phenylalkynyl of 8 to 12 carbon atoms which is unsubstituted or substituted in the phenyl ring by halogen, cyano, nitro or alkyl, $R^3$ is alkyl of 1 to 4 carbon atoms, and Y is oxygen or sulfur.

10. A process for combating the growth of unwanted plants, wherein the plants and/or the soil are treated with a herbicidally effective amount of a 2H-1,2,4,6-thiatriazine-1,1-dioxide of the formula I as claimed in claim 1.

**Revendications**

1.- 1,2,4,6-Thiatriazine-1,1-dioxydes de la formule

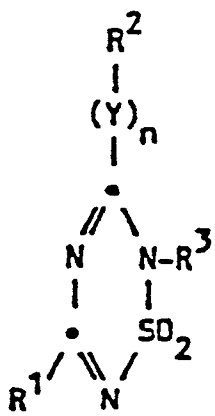

(I),

dans laquelle

$R^1$ désigne un atome d'hydrogène, un radical alkyle ou alcényle non ramifié ou ramifié, comprenant jusqu'à dix atomes de carbone, un radical propargyle, but-1-yne-3-yle ou but-2-ynyle, un groupe aliphatique saturé, ramifié ou non, comportant jusqu'à dix atomes de carbone et halo-substitué, un groupe methoxy-2 éthyle, éthoxy-2 éthyle, méthoxy-3 n-propyle, méthoxy-2 isopropyle, méthoxy-3 n-butyle, méthoxy-1 butyle secondaire, méthoxybutyle tertiaire, éthoxy-butyle tertiaire, méthoxy-2 n-butyle, méthoxy-4 n-butyle, 2-méthyl-mercapto-éthyle, 2-éthyl-mercapto-éthyle, 3-méthyl-mercapto-n-propyle, 3-méthyl-mercapto-n-butyle, 1-méthyl-mercapto-butyle secondaire, méthyl-mercapto-butyle tertiaire ou 2-méthyl-mercapto-n-butyle, un groupe mono-alkyl- ou dialkyl-amino avec un ou des radicaux alkyle en $C_1$ à $C_6$, un groupe cycloalkyle en $C_3$ à $C_7$, un groupe phényle éventuellement substitué par de l'halogène ou des radicaux alkyle ou alcoxy en $C_1$ à $C_4$, un groupe benzyle éventuellement halo-substitué ou un groupe de la formule $R^4$-X-, dans laquelle X représente un atome

d'oxygène ou de soufre ou un groupe -SO- ou -$SO_2$- et $R^4$ possède les significations definies pour $R^1$ à l'exception des groupes mono-alkyl- et dialkyl-amino;

$R^2$ désigne un groupe aliphatique ramifié ou non, saturé ou non saturé, comportant jusqu'à dix atomes de carbone, cyano-, thiocyano-, nitro- ou vinylcéto-substitué, un groupe 2-allyloxy-éthyle, 2-propargyloxy-éthyle, 2-allyloxy-n-propyle, 2-propargyloxy-n-propyle, 3-allyloxy-n-propyle, 4-allyloxy-but-2-ynyle, 2-allylmercapto-éthyle, 2-propargylmercapto-éthyle ou 2-allylmercapto-n-propyle, un groupe alkylène-glycol-alkyle,-alcényle ou -alcynyle comprenant jusgu'à six atomes de carbone et substitué par des radicaux alkyle, alcényle, ou alcynyle comportant jusqu'à quatre atomes de carbone, un groupe alcenyle ou alcynyle avec jusqu'à quatre atomes de carbone, substitué par des groupes alcoxycarbonyle ou alkylmercaptocarbonyle avec jusqu'à cinq atomes de carbone, un groupe alkylidène-amino ou dialkyl-méthylidène-amino avec jusqu'à six atomes de carbone ou un groupe phénylalkyle ou phényl-alcényle ou phényl-alcynyle en $C_8$ à $C_{12}$, éventuellement substitué sur le noyau phényle par des atomes d'halogène, des radicaux alkyle, halo-alkyle, alcoxy, halo-alcoxy, alkylmercapto ou halo-alkyl-mercapto avec jusqu'à quatre atomes de carbone ou des groupes nitro, cyano ou thiocyano;

$R^3$ désigne un atome d'hydrogène, un radical alkyle ou alcényle ramifié ou non, comprenant jusqu'à dix atomes de carbone, un groupe propargyle, but-1-yne-3-yle ou but-2-ynyle, un groupe aliphatique saturé, ramifié ou non, comprenant jusqu'à dix atomes de carbone et halo-substitué, un groupe 2-méthoxy-éthyle, 2-éthoxy-éthyle, 3-méthoxy-n-propyle, 2-méthoxy-isopropyle, 3-méthoxy-n-butyle, 1-méthoxy-butyle secondaire, méthoxy-butyle tertiaire, éthoxybutyle tertiaire, 2-méthoxy-n-butyle ou 4-méthoxy-n-butyle ou un groupe cycloalkyle en $C_3$ à $C_7$;

Y représente un atome, d'oxygène ou de soufre ou un groupe -SO- ou -$SO_2$- et

n = 1, ou,

lorsque $R^1$ = $R^4$ -X- et n = 1,

$R^2$ désigne un groupe benzyle nitro- et(ou) halo-substitué ou un groupe phénoxy-alkyle ou phénoxy-alcényle ou phénoxy-alcynyle en $C_8$ à $C_{12}$, substitué sur le noyau phényle par des halogènes ou des radicaux alkyle, halo-alkyle, alcoxy, halo-alcoxy, alkyl-mercapto ou halo-alkyl-mercapto avec jusqu'à quatre atomes de carbone ou des groupes nitro, cyano ou thiocyano, ou

$R^2$ représente un groupe dialkyl-phosphono et n = O.

2.- 1,2,4,6-Thiatriazine-1,1-dioxyde de la formule I selon la revendication 1, caractérisé en ce que

$R^1$ = $R^4$-X- avec X = O et $R^4$ = alkyle en $C_1$ à $C_4$,

$R^2$ = dialkyl(en $C_1$ à $C_4$)-phosphono,

$R^3$ = alkyle en $C_1$ à $C_4$ et n = O.

3.- 1,2,4,6-Thiatriazine-1,1-dioxydes de la formule I selon la revendication 1, caractérisé en ce que

$R^1$ = $R^4$-X- avec X = O ou S et $R^4$ = alkyle en $C_1$ à $C_4$,

$R^2$ = phénoxy-alkyle en $C_7$ à $C_{10}$ éventuellement halosubstitué sur le noyau phényle,

$R^3$ = alkyle en $C_1$ à $C_4$ et

Y = O ou S.

4.- 1,2,4,6-Thiatriazine-1,1-dioxydes de la formule I selon la revendication 1, caractérisé en ce que

$R^1$ et $R^3$ = alkyle en $C_1$ à $C_4$,

$R^2$ = groupe aliphatique saturé ou non avec jusqu'à quatre atomes de carbone et cyano-substitué et

Y = O ou S.

5.- 1,2,4,6-Thiatriazine-1,1-dioxydes de la formule I selon la revendication 1, caractérisé en ce que

$R^1$ = $R^4$-X- avec X = O ou S et $R^4$ = alkyle en $C_1$ à $C_4$,

$R^2$ = phényl-alkyle, -alcényle ou -alcynyle en $C_8$ à $C_{12}$ éventuellement halo-, cyano-, nitro- ou alkylsubstitué sur le noyau phényle,

$R^3$ = alkyle en $C_1$ à $C_4$ et

Y = O ou S.

6.- Procédé de préparation de 2H-1,2,4,6-thiatriazine-1,1-dioxydes de la formule I selon la revendication 1, caractérisé en ce que l'on fait réagir un 1,2,4,6-thiatriazine-1,1-dioxyde de la formule

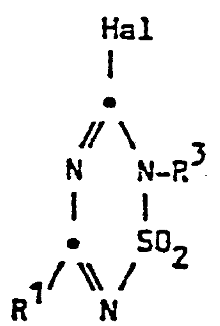

(II),

dans laquelle $R^1$ et $R^3$ possèdent les significations definies plus haut et Hal représente un atome d'halogène, soit avec un composé de la formule

$$H - (Y)_n - R^2 \qquad (III),$$

dans laquelle Y, $R^2$ et n possèdent les significations définies plus haut,

soit avec un sel de métal alcalin ou alcalino-terreux ou d'ammonium d'un composé de la formule III, éventuellement dans un solvant organique inerte et(ou) en présence d'un accepteur (fixateur) d'acide.

7.- Composition herbicide, contenant un 1,2,4,6-thiatriazine-1,1-dioxyde de la formule I selon la revendication 1

8.- Composition herbicide, contenant un 1,2,4,6-thiatriazine, 1,1-dioxyde de la formule I selon la revendication 1 et des additifs inertes.

9.- Composition herbicide, contenant un 1,2,4,6-thiatriazine-1,1-dioxyde de la formule I selon la revendication l'pour lequel $R^1 = R^4$-X- avec $R^4$ = alkyle en $C_1$ à $C_4$ et X = O ou S, $R^2$ = phényl-alkyle,-alcényle ou -alcynyle en $C_8$ à $C_{12}$, éventuellement halo-, cyano-, nitro- ou alkyl-substitué sur le noyau phényle, $R^3$ alkyle en $C_1$ à $C_4$ et Y = O ou S.

10.- Procédé pour combattre le développement de plantes indésirables, caractérisé en ce que l'on traite les plantes ou(et) le sol avec une quantité efficace du point de vue herbicide d'un 2H-1,2,4,6-thiatriazine-1,1-dioxyde de la formule I selon la revendication 1.